# EUROPEAN PATENT APPLICATION

(11) **EP 3 760 204 A1**
(43) Date of publication of application: **06.01.2021**
(21) Application number: 19184228.5
(22) Date of filing: 03.07.2019
(51) Int. Cl.: A61K 31/496, A61K 31/122, A61K 31/4375, A61K 45/06, A61K 9/00, A61P 25/00, A61P 43/00

(54) **MEDICAMENT FOR PREVENTION OR TREATMENT OF HUNTINGTON'S DISEASE**

(71) Applicant: Medizinische Universität Graz, 8036 Graz (AT)
(72) Inventor: WAGNER, Martin, 8010 Graz (AT); PANZITT, Katrin, 8010 Graz (AT)
(74) Representative: Sonn & Partner Patentanwälte

(57) **Abstract**

The present invention provides a pharmaceutical composition for use in prevention or treatment of Huntington's disease in a human. The composition comprises a pregnane X receptor (PXR) ligand selected from rifampicin and hyperforin. Furthermore, pharmaceutical compositions and sets suitable for treating Huntington's disease are provided.

## Description

### Background

Huntington's disease (HD) is a progressive neurodegenerative disorder caused by mutations in the Huntingtin gene, which codes for the protein Huntingtin (HTT; see e.g. Landles, Christian, and Gillian P. Bates. "Huntingtin and the molecular pathogenesis of Huntington's disease: Fourth in Molecular Medicine Review Series." EMBO reports 5.10 (2004): 958-963). Typically, expansion of cytosine-adenine-guanine (CAG) triplet repeats in this gene results in an altered protein (sometimes called mutant Huntingtin; mHTT), which is responsible for HD pathology.

mHTT may be more prone to cleavage creating protein fragments, which have a propensity to undergo misfolding an aggregation. These aggregates have toxic effects in the brain, similar to other protein deposition diseases.

The earliest symptoms are often subtle problems with mood or mental abilities. Motor symptoms typically appear as the disease advances, including a general lack of coordination and advancing to progressively uncoordinated body movements and chorea. Mental abilities generally decline into dementia.

HD can be diagnosed by genetic testing, which can be carried out at any time, regardless of whether or not symptoms are present.

There is no known cure for HD. Treatment options are very limited and generally focus on relieving symptoms. Tetrabenazine may be used for treating chorea associated with HD. Other drugs used include antipsychotics and benzodiazepines.

It is an object of the present invention to provide new options for prevention or treatment of HD.

### Summary of the invention

Therefore, the present invention provides a pharmaceutical composition for use in prevention or treatment of Huntington's disease. The composition comprises a pregnane X receptor (PXR) ligand selected from rifampicin and hyperforin.

In another aspect, the present invention provides a set comprising a first pharmaceutical composition comprising a PXR ligand selected from the group consisting of rifampicin and hyperforin, and a second pharmaceutical composition comprising an additional active agent, preferably tetrabenazine; preferably wherein the set is for prevention or treatment of HD.

In a further aspect, the present invention provides a pharmaceutical composition comprising a PXR ligand selected from the group consisting of rifampicin and hyperforin, and an additional active agent; preferably tetrabenazine, preferably wherein the pharmaceutical composition is for prevention or treatment of HD.

In yet another aspect, the present invention provides a method for delaying the onset of or treating Huntington's disease, comprising
- obtaining a pharmaceutically acceptable formulation comprising a PXR ligand selected from rifampicin and hyperforin; and
- administering an effective amount of the formulation to an individual having Huntington's disease or at risk of developing Huntington's disease.

All embodiments of the invention are described together in the following detailed description and all preferred embodiments relate to all embodiments and aspects alike. All detailed descriptions, e.g. of compositions or modes of administration, relate to preferred embodiments of all aspects of the invention, e.g. compositions for use, sets and methods. All embodiments can be combined with each other, except where stated otherwise.

### Detailed description

The present invention is based on the surprising discovery of a novel signalling pathway for regulating autophagy. It was found that the pregnane X receptor (PXR), also known as the steroid and xenobiotic sensing nuclear receptor or nuclear receptor subfamily 1, group I, member 2, can stimulate autophagy both directly and via the lyosomal biogenesis factor transcription factor EB (TFEB). Stimulating autophagy can enhance the elimination of accumulated protein aggregates, making PXR an attractive new drug target for disorders related to protein accumulation, such as Huntington's disease.

Autophagy is a cellular process which is one of the main mechanisms for the clearance of accumulated misfolded proteins. It has therefore been investigated extensively as a therapeutic target for the treatment of disorders involving protein accumulation (see e.g. Metcalf, Daniel J., et al. "Autophagy and misfolded proteins in neurodegeneration." Experimental neurology 238.1 (2012): 22-28). However, since the regulation of autophagy is very complex, there has been a lack of suitable drug targets.

PXR is a transcription factor that has a role in sensing the presence of foreign toxic substances and in regulating their metabolism. Several agonists of PXR are known and clinically tested, including rifampicin, hyperforin. In the course of the present invention it has been surprisingly found that PXR can stimulate autophagy, making known PXR ligands a new treatment option for diseases involving protein accumulation, such as HD.

Rifampicin (see e.g. Howard, Paul, et al. "Rifampin (INN rifampicin)." Journal of pain and symptom management 50.6 (2015): 891-895), also known as rifampin, is an antibiotic that has been widely used for a long time to treat several types of bacterial infections. Hyperforin (see e.g. Beerhues, Ludger. "Hyperforin." Phytochemistry 67.20 (2006): 2201-2207) is a phytochemical produced e.g. by St John's wort (Hypericum perforatum), which can also be synthesized chemically (Sparling, Brian A., David C. Moebius, and Matthew D. Shair. "Enantioselective total synthesis of hyperforin." Journal of the American Chemical Society 135.2 (2012): 644-647). It has been proposed that hyperforin may be an active agent responsible for the antidepressant and anxiolytic properties of the extracts of St John's wort.

Autophagy is a major degradation pathway for the aggregateprone, disease-causing mHTT in HD. It is therefore well established in the art that HD may be treated by enhancing autophagy (see e.g. Sarkar, Sovan, and David C. Rubinsztein. "Huntington's disease: degradation of mutant huntingtin by autophagy." The FEBS journal 275.17 (2008): 4263-4270). Moreover, it is recognized in the art that enhancing the autophagy-lysosomal pathway is an attractive treatment option for neurodegenerative diseases in general and HD in particular (see e.g. Martini-Stoica, Heidi, et al. "The autophagy-lysosomal pathway in neurodegeneration: a TFEB perspective." Trends in neurosciences 39.4 (2016): 221-234). Especially TFEB has been suggested as a suitable target, being a potent activator of the autophagy-lysosomal pathway by coordinating autophagy induction with lysosomal biogenesis, and its activation has led to amelioration in mouse models of various neurodegenerative disorders. However, there is still a lack of suitable therapeutic options to enhance autophagy.

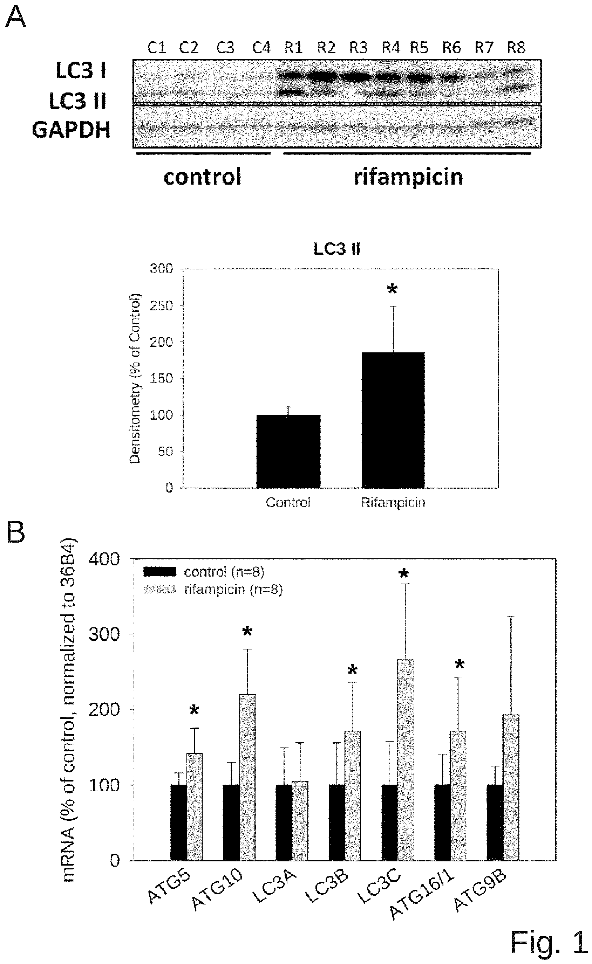

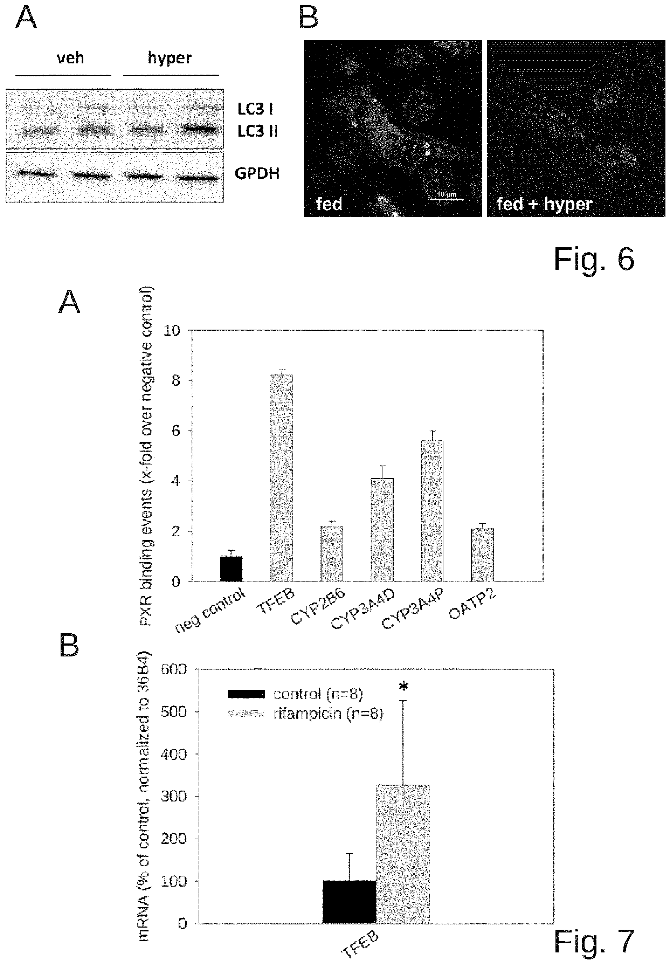

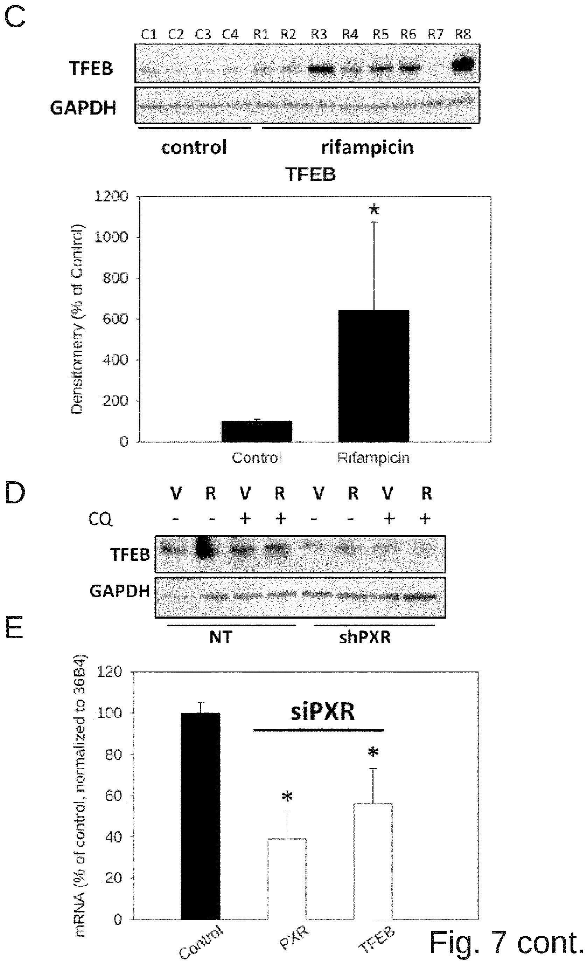

In the course of the present invention, it was demonstrated that, surprisingly, PXR agonists such as rifampicin and hyperforin robustly enhance autophagy (see Example 1 and Figures 1 - 6). Importantly, it was also shown that PXR is expressed in the human brain, making it a suitable therapeutic target for increasing autophagy in the brain (see Figure 12). Both rifampicin (see e.g. Mindermann, Thomas, Werner Zimmerli, and Otmar Gratzl. "Rifampin concentrations in various compartments of the human brain: a novel method for determining drug levels in the cerebral extracellular space." Antimicrobial agents and chemotherapy 42.10 (1998): 2626-2629) and hyperforin (see e.g. Keller, Jan-Henning, et al. "Determination of hyperforin in mouse brain by high-performance liquid chromatography/tandem mass spectrometry." Analytical chemistry 75.22 (2003): 6084-6088.) readily pass the blood-brain-barrier and reach the brain, making them exceptionally suitable for use in the inventive therapy. Moreover, it was surprisingly found that PXR agonists activate TFEB (see Example 1 and Figures 7 - 9), making them particularly well suited for the treatment of HD.

Altogether independently from PXR or autophagy, rifampicin has previously been investigated for its potential brain protective function in Alzheimer's disease or Parkinson's disease (Yulug, Burak, et al. "RIFAMPICIN: an antibiotic with brain protective function." Brain research bulletin 107 (2014): 37-42).

However, prior to the present invention, rifampicin has been regarded as not being suitable for treating HD. In particular, Heiser et al. (Heiser, Volker, et al. "Inhibition of huntingtin fibrillogenesis by specific antibodies and small molecules: implications for Huntington's disease therapy." Proceedings of the National Academy of Sciences 97.12 (2000): 6739-6744) disclose that rifampicin has little or no inhibitory effect on huntingtin aggregation in vitro, thereby in fact teaching away from the present invention. However, aggregation was merely investigated in vitro using purified proteins, making it impossible to observe the PXR and TFEB-mediated therapeutic effects of rifampicin found in the context of the present invention. Im et al. (Im, Wooseok, et al. "Multidrug resistance protein 1 reduces the aggregation of mutant huntingtin in neuronal cells derived from the Huntington's disease R6/2 model." Scientific reports 5 (2015): 16887) disclose that while rifampicin somewhat reduced mHTT accumulation, it did not improve HD disease progression in mice, further strengthening the prejudice that rifampicin is not suitable for the treatment of HD and teaching away from the present invention.

St. John's wort extract(Hypericum perforatum), which contains hyperforin as one of its active ingredients, has been investigated and is used for treating depression and mood disorders. Some studies in healthy rodents have suggested that St. John's Wort extract has potential negative effects on prepulse inhibition and should therefore not be used by HD patients who suffer from prepulse inhibition deficit (Klemow, Kenneth M., et al. "11 Medical Attributes of St. John's Wort (Hypericum perforatum)." Lester Packer, Ph. D. (2011): 211; Tadros, Mariane G., et al. "Proapoptotic and prepulse inhibition (PPI) disrupting effects of Hypericum perforatum in rats." Journal of ethnopharmacology 122.3 (2009): 561-566), thereby teaching away from the present invention. However, these tests were only carried out in healthy rodents with high doses of St. John's Wort extract and it is not clear if the potential negative effects observed came from hyperforin or from other components of the extract. St. John's wort has been widely used for depression and is recognized as safe for use in humans (Bilia, Anna Rita, Sandra Gallori, and Franco F. Vincieri. "St. John's wort and depression: efficacy, safety and tolerability-an update." Life sciences 70.26 (2002): 3077-3096). In any case, prior to the present invention, it was not known that hyperforin could be used to treat HD.

In a preferred embodiment, the invention provides a combination therapy for treating HD comprising a PXR ligand and a further active agent. Preferably the further active agent is an active agent for treating symptoms associated with HD, in particular tetrabenazine. The combination therapy according to the invention is particularly advantageous since the additional active agent targets the symptoms associated with HD (e.g. chorea), whereas the PXR ligand can target actual disease-causing factors.

The treatment of symptoms associated with HD is well known in the art (see e.g. Adam, Octavian R., and Joseph Jankovic. "Symptomatic treatment of Huntington disease." Neurotherapeutics 5.2 (2008): 181-197), though not in connection with PXR ligands. In the context of the present invention, the further active agent may e.g. be any active agent commonly used for the treatment of symptoms associated with HD. In particular the further active agent may be an antipsychotic, preferably an antipsychotic selected from the group consisting of haloperidol, chlorpromazine, risperidone and quetiapine. In a further preferred embodiment the further active agent is a benzodiazepine. It a further preferred embodiment, the further active agent is selected from the group consisting of sulpiride, tiapride, thiopropazate, haloperidol, clozapine, tetrabenazine, amantadine, riduzole, coenzyme Q10, remacemide, lamotrigine, donepezil, fluoxetine, levetiracetam, and clonazepam. In the context of the present invention it is most preferred if the additional active agent is tetrabenazine.

In a preferred embodiment, the composition for use according to the present invention therefore further comprises at least one excipient and/or at least one additional active agent, preferably wherein the additional active agent is tetrabenazine.

A further preferred embodiment of the present invention relates to the composition for use according to the invention, wherein the PXR ligand is administered to a subject, and wherein the additional active agent is administered to the subject in a composition together with the PXR ligand, in combination with the PXR ligand, before the PXR ligand is administered, or after the PXR ligand has been administered.

In another embodiment, the PXR ligand is the single active agent in the pharmaceutical composition (preferably in the presence of one or more excipients).

The subject to be treated according to the present invention is a human. The subject preferably has or is predisposed to any one of the diseases and conditions mentioned herein.

The term "preventing" or "prevention" as used herein means to stop a disease state or condition from occurring in an individual completely or almost completely or at least to a (preferably significant) extent, especially when the individual is predisposed to such a risk of contracting a disease state or condition. However, these terms should not be interpreted as an absolute success in the sense that a subject can never develop an associated disease, reaction or condition but as the reduction of the chance of developing the disease, reaction or condition in a prophylactic treatment.

In the context of the present invention, the subject is preferably known to be at risk of developing HD, e.g. because of genetic risk factors, family history or other risk factors.

Typically, HD is a gradually progressive disorder, implying that there is a period during which the disease has started, but definitive clinical symptoms sufficient for diagnosis have not yet appeared. The earlier phase of the disease in which no clinical symptoms are present but a disease-specific pathology (e.g. a genetic mutation) is present is typically referred to as the preclinical phase. During the preclinical phase, the disease can already be diagnosed, e.g. through genetic tests or other molecular tests.

The term "clinical symptom" as used herein, is to be understood as a manifestation of the disease that is apparent to the subject, e.g. lack of coordination or chorea.

In a preferred embodiment of the invention, the HD is in the preclinical phase. According to a preferred embodiment, the subject does not yet present clinical symptoms associated with HD.

In a further embodiment, the subject has a (disease-causing) mutation in the Huntingtin gene (NCBI Entrez Gene GeneID 3064 for H. sapiens). Preferably the mutation is a mutation that causes HD. In an especially preferred embodiment, the subject has a Huntingtin gene with a cytosine-adenine-guanine (CAG)-repeat count of at least 27, preferably at least 33, even more preferably at least 36, especially at least 38, yet even more preferably at least 40, most preferably at least 42. The trinucleotide (CAG) repeat count refers to the number of CAG repeats located in the 5' region of the Huntingtin gene. The number of CAG repeats has been shown to be predictive of the clinical features of HD (see e.g. Andrew, Susan E., et al. "The relationship between trinucleotide (CAG) repeat length and clinical features of Huntington's disease." Nature genetics 4.4 (1993): 398).

In a further preferred embodiment, the subject is a human having an age below 70 years, more preferably below 60 years, even more preferably below 50 years, yet even more preferably below 40 years and/or a human who had HD onset before the age of 70 years, preferably before the age of 60 years, even more preferably below the age of 50 years, yet even more preferably below the age of 40 years.

In order to prevent or delay disease progression it is preferred if the treatment is continued for an extended period of time. Accordingly, in a preferred embodiment, the inventive composition, set or combination therapy is administered to the subject over a period of at least 2 weeks, preferably at least 4, more preferably at least 8, even more preferably at least 12, yet even more preferably at least 16 or even at least 20, especially at least 26 or even at least 52 weeks.

The present invention also relates to diagnosing a subject with HD or a predisposition thereto and then treating the subject with a composition, set or combination therapy according to the present invention. The diagnosis is not necessarily performed together with the inventive treatment. Said diagnosis can e.g. be a genetic test.

The present invention also relates to a method for preventing or treating HD in a subject comprising the steps of: (1) Identifying a subject having HD or being at risk of developing HD, preferably by a genetic test, in particular wherein the subject has a mutation in a gene associated with an increased risk of developing HD, in particular wherein the gene is HTT; (2) obtaining a PXR ligand selected from the group consisting of rifampicin and hyperforin; and (3) administering an efficient amount of said PXR ligand to the subject.

In the context of the present invention, the expression "pharmaceutical composition" refers to any composition comprising at least one active agent (e.g. the PXR ligand), and preferably one or more excipients, which is pharmaceutically acceptable for administration to an individual, especially a mammal, in particular a human.

In a preferred embodiment, the composition or the composition for use according to the invention is provided for oral administration, preferably wherein the composition is provided as a tablet, a capsule, a suspension or a solution. If the pharmaceutical composition is intended for oral intake, it may also be in the form of pastilles, tablets, troches, lozenges, pills, gums, powders or drinking solutions. Systemic distribution of the PXR ligand can be facilitated by formulations and carriers known in the state of the art.

In a further preferred embodiment, the composition or the composition for use according to the invention is provided for intravenous administration, preferably wherein the composition is provided as a powder for reconstitution (e.g. in a physiologic salt solution) or as a solution (e.g. an isotonic solution with physiological pH).

The formulation or composition may comprise pharmaceutical carriers, excipients, preferably polymeric excipients, or additives. The term "carrier" refers to a diluent, e.g. water, saline, excipient, or vehicle, with which the composition can be administered. For a solid or fluid composition the carriers or additives in the pharmaceutical composition may comprise SiO₂, HO₂, a binder, such as microcrystalline cellulose, polyvinylpyrrolidone (polyvidone or povidone), gum tragacanth, gelatine, starch, lactose or lactose monohydrate, alginic acid, maize (corn) starch and the like; a lubricant or surfactant, such as magnesium stearate, or sodium lauryl sulphate; a glidant, such as colloidal silicon dioxide; a sweetening agent, such as sucrose or saccharin. Preferably, the preparation comprises buffers or pH adjusting agents, e.g. selected from citric acid, acetic acid, fumaric acid, hydrochloric acid, malic acid, nitric acid, phosphoric acid, propionic acid, sulfuric acid, tartaric acid, or combinations thereof. The PXR ligand can be in the form of a pharmaceutically acceptable salt, for example sodium salt of the PXR ligand. Other pharmaceutically acceptable salts include, among others, potassium, lithium and ammonium salts. Preferred excipients are polymers, especially cellulose and cellulose derivatives.

According to a particularly preferred embodiment of the present invention, the pharmaceutical composition is a liquid and preferably an aqueous solution. In general, liquid compositions are especially suitable for intravenous administration. Suitable excipients are known to the person skilled in the art, for example water (especially water for injection), saline, Ringer's solution, dextrose solution, buffers, Hank solution, vesicle forming compounds (e.g. lipids), fixed oils, ethyl oleate, 5% dextrose in saline, substances that enhance isotonicity and chemical stability, buffers and preservatives, such as benzalkonium chloride. The pharmaceutical composition according to the present invention may be liquid or ready to be dissolved in liquid such as sterile, deionised or distilled water, or sterile isotonic phosphate-buffered saline (PBS). Preferably, 1000 µg (dry-weight) of such a composition comprises or consists of 0.1-990 µg, preferably 1-900µg, more preferably 10-200µg PXR ligand, and optionally 1-500 µg, preferably 1-100 µg, more preferably 5-15 µg (buffer) salts (preferably to yield an isotonic buffer in the final volume), and optionally 0.1-999.9 µg, preferably 100-999.9 µg, more preferably 200-999 µg other excipients. Preferably, 100 mg of such a dry composition is dissolved in sterile, deionised/distilled water or sterile isotonic phosphate-buffered saline (PBS) to yield a final volume of 0.1-100 ml, preferably 0.5-20 ml, more preferably 1-10 ml. Preferably, the concentration of the PXR ligand is 0.01 mM - 1000 mM, preferably 0.1 mM - 500 mM, more preferably 0.25 mM - 250 mM, even more preferably 0.5 mM - 100 mM, in particular 1 mM - 50 mM or even 2.5 mM - 25 mM.

It is evident to the skilled person that active agents and drugs described herein can also be administered in salt form (i.e. as a pharmaceutically acceptable salt of the active agent). Accordingly, any mention of an active agent herein shall also include any pharmaceutically acceptable salt forms thereof.

In a preferred embodiment, a dose of the composition is administered to a subject. It is preferred that the dose contains between 10 mg and 1000 mg, preferably between 50 and 1000 mg, more preferably between 100 mg and 1000 mg, even more preferably between 200 mg and 800 mg, most preferably between 400 mg and 600 mg, in particular 600 mg rifampicin. In another preferred embodiment, the dose is between 0.1 mg/kg body weight of a patient and 50 mg/kg, preferably between 0.4 mg/kg and 25 mg/kg, even more preferably between 2 mg/kg and 15 mg/kg, most preferably between 5 mg/kg and 10 mg/kg. In a further preferred embodiment, a dose of the composition is administered to a subject, wherein the dose contains between 0.05 mg and 500 mg, preferably between 0.2 and 200 mg, more preferably between 1 mg and 50 mg, even more preferably between 4 mg and 20 mg hyperforin. In another preferred embodiment, the dose is between 0.001 mg/kg body weight of a patient and 40 mg/kg, preferably between 0.01 mg/kg and 10 mg/kg, even more preferably between 0.05 mg/kg and 4 mg/kg, most preferably between 0.1 mg/kg and 1 mg/kg.

It is further preferred, if the dose is administered at least once per week, preferably at least three times per week, even more preferably at least once per day, yet even more preferably at least twice per day, in particular three times per day.

The present invention is further illustrated by the following figures and examples, without being restricted thereto.
**Figure 1****. The PXR ligand rifampicin induces markers of autophagy on protein and mRNA levels in human liver biopsy samples invivo.** Liver biopsy samples from individual patients receiving either placebo (control C1-C4, n=4) or rifampicin R1-R8, n=8) have been analyzed by Western blotting (A) and qPCR (B) for markers of autophagy. A. Rifampicin significantly induces the autophagy readout marker LC3 II. Densitometry of Western blot data for LC3 II are shown on the bottom. B. RT-qPCR of selected autophagy genes in control and rifampicin-treated patients (n=8 each) shows significant induction of ATG5, ATG10, LC3B/C and ATG16/1. * p<0.05, unpaired t-test.

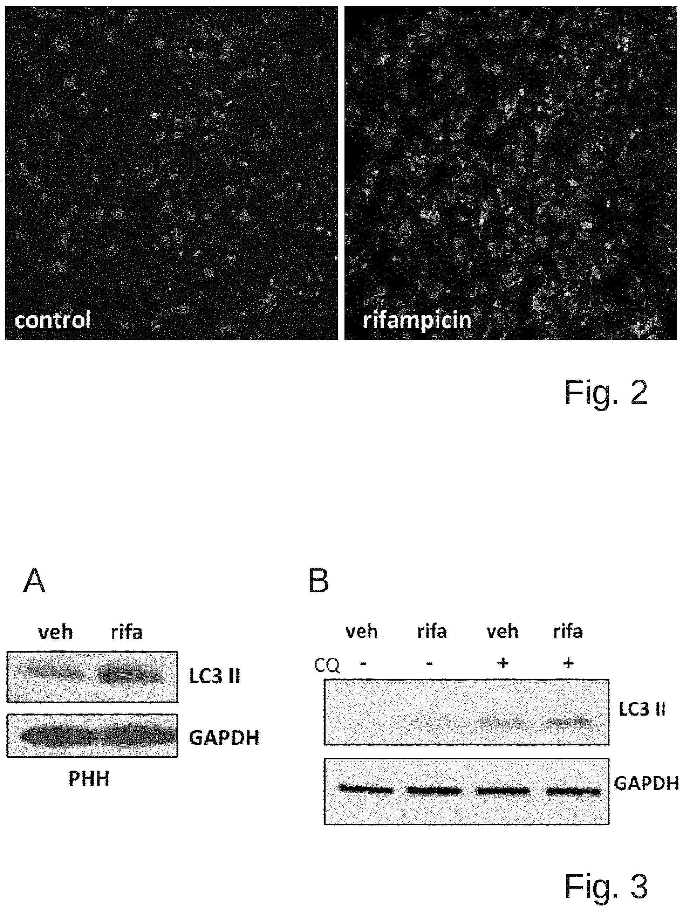

**Figure 2****. The PXR ligand rifampicin induces autophagic flux in human liver biopsy samples in vivo.** Liver biopsy samples from individual patients receiving either placebo (control) or rifampicin has been co-stained for dapi (blue, nuclei), LC3A (red, autophagosomes) and cathepsin D (green, lysosomes). Rifampicin significantly increases fusion of autophagosomes with lysosomes indicated by an increased merging orange signal. A representative picture is shown; magnification, 40x.

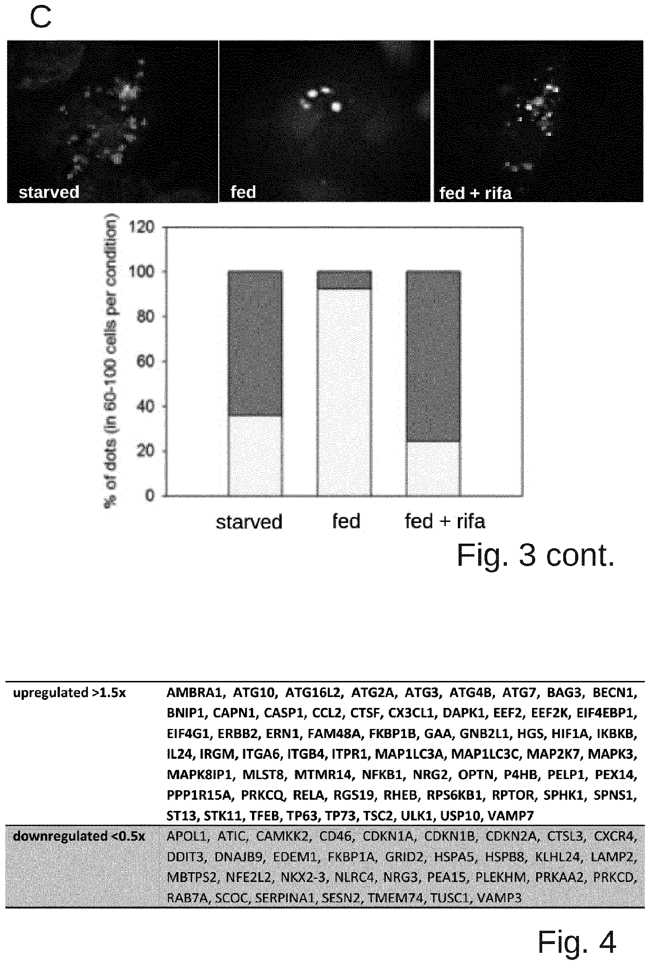

**Figure 3****. The PXR ligand rifampicin induces autophagic flux invitro.** Primary human hepatocytes (PHH) (A) and HepG2 cells (B, C) were treated with 50µM rifampicin (rifa) or ethanol as vehicle (veh). Autophagic flux assays was performed in HepG2 cells according the "Guidelines for the use and interpretation of assays for monitoring autophagy" (Klionsky DJ, Abdelmohsen K, Abe A, Abedin MJ, Abeliovich H, Acevedo Arozena A, Adachi H, et al. Guidelines for the use and interpretation of assays for monitoring autophagy (3rd edition). Autophagy 2016;12:1-222.). A. Rifampicin induces LC3 II protein of primary human hepatocytes (pooled duplicates of two different batches of PHH). B. Rifampicin induces LC3 II protein in the presence of the lysosomal inhibitor chloroquine (CQ) indicative of increased autophagic flux (pooled triplicates). Of note, in contrast to liver tissue, HepG2 cells display a prominent LC3 II band and only weak LC3 I. C. HepG2 cells were transfected with pGFP-RFP-LC3. Suppressed autophagy (fed condition) results in yellow dots as a merge of GFP and RFP fluorescence (middle). Induction of autophagy results in breakdown of GFP, resulting in red dots from the left-over RFP fluorescence indicating induced autophagic flux (starved condition) (left). Rifampicin increases autophagic flux in fed cells (right). Quantification by dot counting in 60-100 cells per condition (bottom panel).
**Figure 4****. Re-analysis of a RNA Seq data set focusing on autophagy-related genes.** Re-analysis of a published RNA Seq data set from rifampicin-treated human primary hepatocytes (Smith RP, et al. PLoS Genet 2014;10:e1004648). From a list of 231 autophagy-related genes 65 genes were upregulated > 1.5 fold and 34 genes downregulated < 0.5 fold.

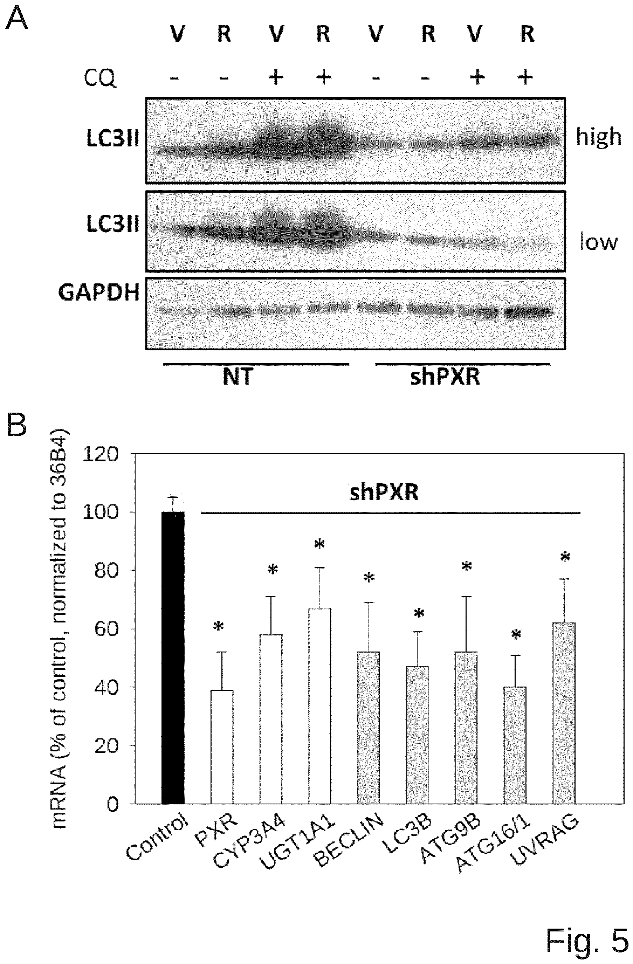

**Figure 5****. Rifampicin induces autophagy flux PXR dependently.** A. PXR was silenced using shRNA (shPXR) in HepG2 cells, non-targeting siOligos were used as control (NT). HepG2 cells were then treated with 50µM rifampicin (R) or ethanol as vehicle (V) in the absence or presence of the lysosomal inhibitor chloroquin (CQ). In shPXR HepG2 cells, chloroquine treatment leads to a markedly reduced accumulation of LC3 II (high and low exposure) indicating reduced basal autophagy. Induction of autophagic flux by rifampicin (R) is blocked in siPXR cells indicating PXR-dependent effects. B. mRNA expression levels of PXR, classical PXR target genes (white) and autophagy-related genes (gray) are significantly reduced in siPXR HepG2 cells compared to NT control HepG2 cells (black bar).
**Figure 6****. Hyperforin induces autophagy flux.** A. HepG2 cells were treated with 50µM hyperforin (hyper) or ethanol as vehicle (veh). Autophagic flux assays was performed in HepG2 cells according the "Guidelines for the use and interpretation of assays for monitoring autophagy" (Klionsky DJ, Abdelmohsen K, Abe A, Abedin MJ, Abeliovich H, Acevedo Arozena A, Adachi H, et al. Guidelines for the use and interpretation of assays for monitoring autophagy (3rd edition). Autophagy 2016;12:1-222.). A. Hyperforin induces LC3 II protein in HepG2 cells (pooled triplicates). C. HepG2 cells were transfected with pGFP-RFP-LC3. Suppressed autophagy (fed condition) results in yellow dots as a merge of GFP and RFP fluorescence (left). Induction of autophagy results in breakdown of GFP, resulting in red dots from the left-over RFP fluorescence indicating induced autophagic flux in hyperforin treated fed HepG2 cells (right).
**Figure 7****. TFEB is a direct PXR target and induced by rifampicin.** A. PXR binding site in the first intron of TFEB retrieved from PXR-ChIP Seq analysis and evaluated by PXR ChIP-PCR along with established PXR binding sites in a human liver biopsy invivo. B/C. Liver biopsy samples from individual patients receiving either placebo (control C1-C4, n=4) or rifampicin R1-R8, n=8) have been analyzed by qPCR and Western blotting for TFEB expression. Rifampicin significantly induces TFEB on mRNA (B) and protein levels (C) invivo. Densitometry of Western blot data for TFEB are shown on the bottom of (C). D/E. PXR was silenced using shRNA (shPXR) in HepG2 cells, non-targeting siOligos were used as control (NT). HepG2 cells were then treated with 50µM rifampicin (R) or ethanol as vehicle (V) in the absence or presence of the lysosomal inhibitor chloroquin (CQ). In shPXR HepG2 cells protein expression of TFEB (D) is significantly abolished and not inducible by rifampicin anymore. PXR knockdown also significantly reduces TFEB mRNA levels (E). * p<0.05, unpaired t-test.

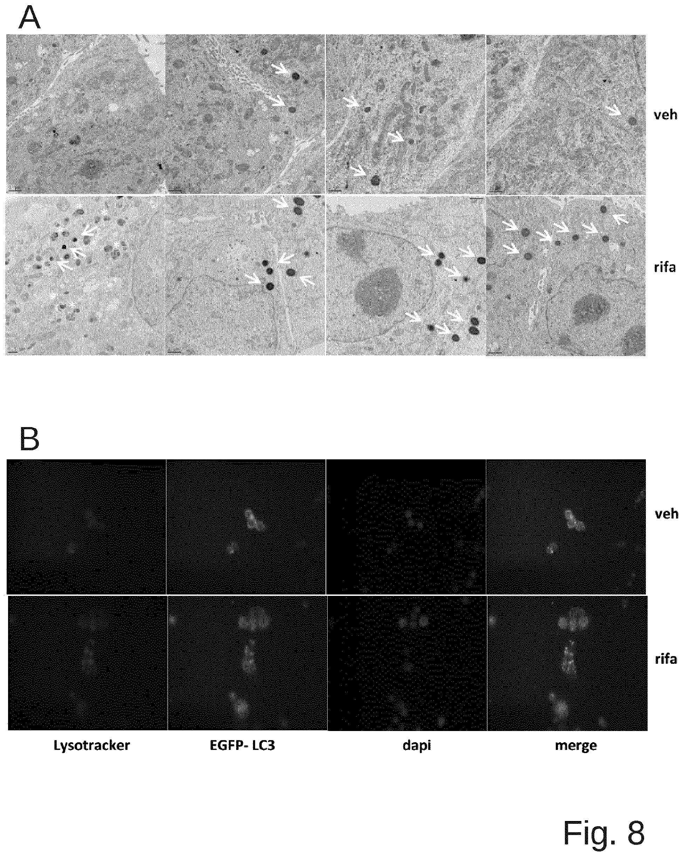

**Figure 8****. Rifampicin increases lysosomal bioformation.** HepG2 cells were treated with 50µM rifampicin (rifa) or ethanol as vehicle (veh) and examined under fed conditions. A. Electronmi-croscopy reveals significantly more lysosmes (arrows) and autophagolysomoses with breakdown products (asterixs) in rifampicin treated cells. B. Immunoflourescence with lysotracker (red, lysosmoses), transfected EGFP-LC3 (green, autophagosomes), dapi (blue, nuclei) and yellow merge. Rifampicin increases the number of lysosmomes which readily fuse with autophagosomes.

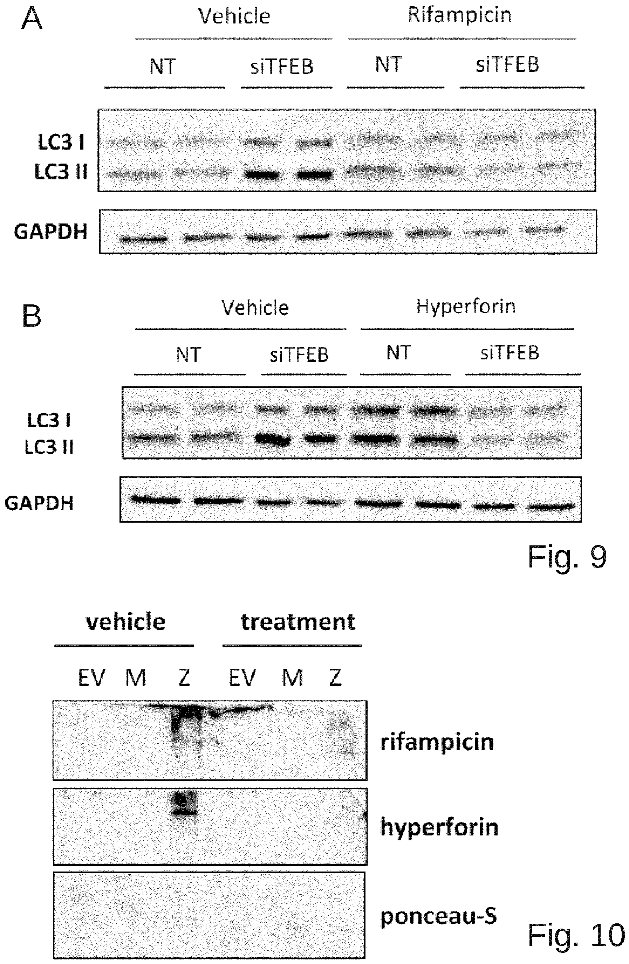

**Figure 9****. TFEB knockdown prevents LC3 induction by PXR ligands.** TFEB was silenced using siRNA (siTFEB) in HepG2 cells, non-targeting siOligos were used as control (NT). HepG2 cells were then treated with 50µM rifampicin (rifa), 50µM hyperforin (hyper) or ethanol as vehicle (veh) in the absence or presence of the lysosomal inhibitor chloroquin (CQ). A. Rifampicin induces LC3 II in the presence of TFEB. LC3 II induction is blunted in siTFEB cells indicating TFEB dependent autophagy induction. B. Hyperforin induces LC3 II in the presence of TFEB. LC3 II induction is blunted in siTFEB cells indicating TFEB dependent autophagy induction.
**Figure 10****. Rifampicin and hyperforin reduce pathological alpha-1-antitrypsin accumulation.** HeLa cells were transfected with PXR/RXR and an empty plasmid (EV) or a plasmid containing either the normal (M) or the pathological Z-allel (Z) of alpha-1-antitrypsin leading to pathological protein accumulation. Cells were then treated for 24h with vehicle (ethanol), 50µM rifampicin or 50µM hyperforin. Rifampicin and hyperforin treatment robustly decreases pathological A1AT accumulation.

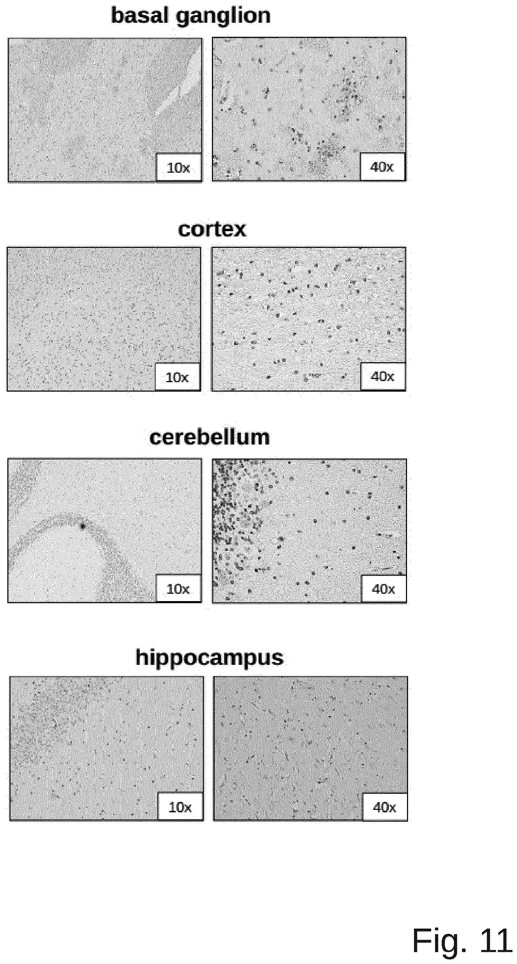

**Figure 11****. PXR is expressed in the human brain.** Tissue sections from basal ganglion, cortex, cerebellum, and the hippocampus. Immunohistochemical staining for PXR confirmed that PXR is expressed in neurons. Therefore, it is highly plausible that autophagy can be stimulated in the brain by activating PXR. Representative pictures are shown; magnification, 10x or 40x as indicated.

### Example 1 - The PXR-TFEB-autophagy signalling pathway is a new therapeutic drug target for the treatment of protein-accumulation-related neurodegenerative diseases such as Huntington's disease

### Materials and Methods

**Cell culture and transfection:** Cells were kept under regular growth conditions (5% CO₂) in DMEM media containing 10% FBS, 1% Penicillin/Streptomycin and 1/100 Na-Pyruvate (all Gibco). Prior to transfections, cells were plated in 6-well dishes at 2x10^5 cells/well. Cells were transfected with GFP-RFP-LC3 (gift from David D Moore, Baylor College of Medicine, Houston, TX, USA) or EGFP-LC3 (Addgene, Watertown, USA) using Lipofectamine 3000 as transfectant as per protocol (Invitrogen). Lysotracker (Thermo Fisher) was used to stain lysosomes according to protocol. After 24h the transfection media was changed to fresh media containing the respective treatment. Cells were treated with either chloroquine[25pM], rifampicin[50pM], hyperforin[1µM], or Vehicle (Ethanol) for another 24h. After that cells were harvested and processed for further investigation.

For siRNA experiments the respective siRNA nucleotides were purchased from Dharmacon (smartpool) and transfected with RNAiMaX (Invitrogen) as per protocol. For shPXR HepG2 cells were transduced with attenuated adenovirus (titer: 10^5/6-well) produced in 293T cells. The respective plasmids were a kind gift of David D Moore, Baylor College of Medicine, Houston, TX, USA.

**RNA preparation, RT qPCR and ChIP PCR:** RNA isolation from human tissue (Marschall HU, Wagner M, Zollner G, Fickert P, Diczfalusy U, Gumhold J, Silbert D, et al. Complementary stimulation of hepatobiliary transport and detoxification systems by rifampicin and ursodeoxycholic acid in humans. Gastroenterology 2005;129:476-485.) and cell lines was performed following the RNeasy protocol (Qiagen, RNeasy Mini kit) including DNAse I digest on column. RNA concentrations were measured by Nanodrop and 1000ng of RNA were subjected to cDNA synthesis (Superscript III, Invitrogen). RT qPCR was performed on a Light cycler (Roche) with Luna Universal qPCR Master Mix (NEB). All RT qPCR data was normalized to the house keeping gene 36B4 and the fold change was calculated with the ΔΔCt method. Ct values >35 were considered as negative.

For ChIP PCR, a ChIP preparation following the Active motif high sensitivity kit's protocol was performed. 3 different binding sites were tested in normal liver tissue based on an in silico PXR response element search. 1 site was significantly occupied by TFEB. This site is located app. 7kb upstream of the first exon of the short variant of TFEB or otherwise in the first intron of the long variant of TFEB in an H3K27Ac active site (UCSC).

**Western blot:** Protein lysate was generated by homogenization (Magnalyzer, Roche) of tissue or cell pellet in homogenization buffer with protease and phosphatase inhibitors (Halt tablets, Invitrogen). After homogenization, lysates were sonicated 2x for 10" each. Lysates were then centrifuged for 10 min at 4°C and the supernatant was subjected to BCA protein estimation (Thermo Fisher). Equal amounts of lysate were loaded to self-made acrylamide gels. After separation, the protein was transferred to a PVDF membrane with semi-dry transfer. Primary antibody incubation (LC3B, Novus; GAPDH, Santa Cruz; TFEB, Abcam) took place over night at 4°C and secondary antibody (anti-rabbit-HRP or anti mouse-HRP) incubation for 1.5-2 h at room temperature. Development of the blots was performed with Clarity reagent (Biorad) on a Biorad developer. For native gels homogenization took place through 1 freeze thaw cycle in homogenization buffer with protease and phosphatase inhibitors (Halt tablets, Invitrogen). All buffers, dyes and gels were prepared without SDS.

**Immunofluorescence (IF) and Immunohistochemistry (IHC):** For IHC tissue sections (4µM for cryo, 2M for paraffin) were cut and processed as per the antibody supplier's protocol for IHC. Briefly, sections were fixed in 4% paraformaldehyde (PFA), antigen retrieval was performed and then the sections were incubated with the primary (LC3A, Cell Signaling; Cadhepsin D, Abcam) and secondary antibody (anti-rabbit Alexa Fluor 488 or anti mouse Alexa Fluor 594) sequentially for 1h at room temperature. Slides were developed with rabbit-on-rodent-HRP-polymer (Biocare Medical). For IF cells were grown on glass chamber slides. They were fixed in 4% PFA, incubated with the primary and secondary antibody sequentially for 1h at room temperature followed by the secondary antibodies in the same way. Slides were mounted in DAPI containing mounting media (Invitrogen) and kept in the dark. Microscopy was performed on a Nikon laser scan microscope.

**Electron Microscopy (EM):** EM was performed using standard protocols. Treated cells fixed in 2% PFA + 2,5% glutaraldehyde in 0.1M NA-phosphate buffer were provided as starting material.

**ChIP-seq re-evaluation:** An already published dataset of Rifampicin treated primary human hepatocytes (Smith RP, Eckalbar WL, Morrissey KM, Luizon MR, Hoffmann TJ, Sun X, Jones SL, et al. Genome-wide discovery of drug-dependent human liver regulatory elements. PLoS Genet 2014;10:e1004648; Bioprojects PRJNA 239635 and 239637) was re-analyzed specifically for its TFEB expression using the galaxy platform for various analyses (https://galaxy.medunigraz.at).

### Results:

In a previous study we have treated patients with the human PXR ligand rifampicin to test whether "classical" phase I, phase II and phase III detoxifying enzymes/transporters are induced in human liver tissue (Marschall HU, et al. Gastroenterology 2005;129:476-485). We now showed that in livers of rifampicin-treated patients LC3 (microtubule-associated protein 1A/1B-light chain 3) II, as a readout marker for autophagy, is significantly increased on protein levels along with several autophagy-related genes (i.e. ATG5, ATG10, LC3B and LC3C) on RNA levels (Figure 1A and 1B). Rifampicin increased autophagy flux in human liver, since autophagosomes and lysosomes increasingly fused to autophagolysosomes under treatment conditions (Figure 2). The effects of autophagy induction by rifampicin were reproduced in cell culture experiments with primary human hepatocytes (Figure 3A) and the liver cell line HepG2 (Figure 3B and 3C) and confirmed increased autophagic flux in co-treatment assays with chloroquine (Figure 3B) and LC3-GFP-RFP co-labeling experiments (Figure 3C). In addition, when re-analyzing a published RNA Seq data set from rifampicin-treated human primary hepatocytes (Smith RP, et al. PLoS Genet 2014;10:e1004648), we found from a list of 231 autophagy-related genes 65 genes upregulated >1.5 fold and 34 genes downregulated <0.5 fold (Figure 4). To test if the observed effects truly depend on PXR we next knocked-down PXR in human hepatocytes and treated cells with rifampicin +/- chloroquine (the latter one commonly used to block autophagic flux (Klionsky DJ, Abdelmohsen K, Abe A, Abedin MJ, Abeliovich H, Acevedo Arozena A, Adachi H, et al. Guidelines for the use and interpretation of assays for monitoring autophagy (3rd edition). Autophagy 2016;12:1-222)). Accumulation of LC3 was significantly impaired already under baseline conditions when autophagic flux was blocked by chloroquine. Moreover, the rifampicin induced increase of autophagy was completely blunted in PXR knockdown cells (Figure 5A). On transcriptional levels, autophagy-related genes such as ATG5, ATG10, RAB7 (not shown), BECLIN, LC3B, ATG9B were significantly reduced in PXR knockdown cells (Figure 5B). Similar effects of autophagy induction were observed with hyperforin, the main active compound of St. John's wort (Figure 6). Of note, we also tested whether activation of the other xenobiotic receptor CAR has any impact on autophagy (using our RFP-GFP-LC3 cell line assay) but could not detect any changes in autophagy activity when cells were treated with the specific human CAR ligand CITCO (not shown).

Mechanistically, the master regulator for lysosomal biogenesis, transcription factor EB (TFEB) (Settembre C, Di Malta C, Polito VA, Garcia Arencibia M, Vetrini F, Erdin S, Erdin SU, et al. TFEB links autophagy to lysosomal biogenesis. Science 2011;332:1429-1433) is a key mediator of the PXR mediated regulation of autophagy. Re-analysis of a published PXR ChIP-Seq data set (Smith RP, et al. PLoS Genet 2014;10:e1004648) showed a robust PXR binding site in the first intron of TFEB, which we could re-capitulate by PXR ChIP PCR in human liver (Figure 7A). Moreover, TFEB was also significantly induced in our human biopsy samples on protein and mRNA levels (Figure 7B and 7C) and vice versa downregulated in the shPXR HepG2 cells on protein and transcript levels (Figure 7D and 7E). In line with the functional properties of TFEB lysosomes were elevated in numbers as demonstrated with electron microscopy (Figure 8A) and lysosomal IF staining (Figure 8B). Most convincingly, knockdown of TFEB significantly abrogated LC3 induction by rifampicin and hyperforin (Figure 9).

Functionally, we tested invitro whether rifampicin and hyperforin are able to reduced aggregates of pathological alpha-1-antitrypsin which typically accumulate in alpha-1-antitrypsin related chronic liver disease. Both, rifampicin and hyperforin robustly reduce pathological alpha-1-antitrypsin aggregates invitro in a human cell line (Figure 10).

Given that a) PXR is expressed in the human brain (Figure 11); b) that both rifampicin and hyperforin readily pass the blood-brain-barrier; c) that the PXR agonists rifampicin and hyperforin robustly enhance autophagy (Figures 1 - 6); d) that autophagy is a major degradation pathway for mHTT and HD may be treated by enhancing autophagy; and e) that enhancing the autophagy-lysosomal pathway through TFEB is particularly beneficial in HD, it is highly plausible that the PXR ligands rifampicin and hyperforin are well suited for the treatment of HD.

## Claims

1. A pharmaceutical composition for use in prevention or treatment of Huntington's disease in a human, the composition comprising a pregnane X receptor (PXR) ligand selected from rifampicin and hyperforin.

2. The composition for use according to claim 1, wherein the composition further comprises at least one excipient and/or at least one additional active agent, preferably wherein the additional active agent is tetrabenazine.

3. The composition for use according to claim 1 or 2, wherein the PXR ligand is administered to a subject, and
wherein an additional active agent is administered to the subject in a composition together with the PXR ligand, in combination with the PXR ligand, before the PXR ligand is administered, or after the PXR ligand has been administered,
preferably wherein the additional active agent is tetrabenazine.

4. The composition for use according to any one of claims 1 to 3, wherein the composition is provided for oral administration, preferably wherein the composition is provided as a tablet, a capsule, a suspension or a solution.

5. The composition for use according to any one of claims 1 to 4, wherein the composition is provided for intravenous administration, preferably wherein the composition is provided as a powder for reconstitution or as a solution.

6. The composition for use according to any one of claims 1 to 5, wherein a dose of the composition is administered to a subject and wherein the dose contains between 10 mg and 1000 mg, preferably between 50 and 1000 mg, more preferably between 100 mg and 1000 mg, even more preferably between 200 mg and 800 mg, most preferably between 400 mg and 600 mg, in particular 600 mg rifampicin.

7. The composition for use according to any one of claims 1 to 6, wherein a dose of the composition is administered to a subject and wherein the dose contains between 0.05 mg and 500 mg, preferably between 0.2 and 200 mg, more preferably between 1 mg and 50 mg, even more preferably between 4 mg and 20 mg hyperforin.

8. The composition for use according to claim 5 or 6, wherein said dose is administered at least once per week, preferably at least three times per week, even more preferably at least once per day, yet even more preferably at least twice per day, in particular three times per day.

9. The composition for use according to any one of claims 1 to 8, wherein the Huntington's disease is in the preclinical phase.

10. The composition for use according to any one of claims 1 to 9, wherein the subject has a Huntingtin gene with a cytosine-adenine-guanine (CAG)-repeat count of at least 27, preferably at least 33, even more preferably at least 36, especially at least 38, yet even more preferably at least 40, most preferably at least 42.

11. The composition for use according to any one of claims 1 to 10, wherein the subject is a human having an age below 70 years, more preferably below 60 years, even more preferably below 50 years, yet even more preferably below 40 years and/or a human who had Huntington's disease onset before the age of 70 years, preferably before the age of 60 years, even more preferably below the age of 50 years, yet even more preferably below the age of 40 years.

12. A set comprising a first pharmaceutical composition comprising a PXR ligand selected from the group consisting of rifampicin and hyperforin, and a second pharmaceutical composition comprising an additional active agent, preferably tetrabenazine.

13. A pharmaceutical composition comprising a PXR ligand selected from the group consisting of rifampicin and hyperforin, and an additional active agent, preferably tetrabenazine.

14. Pharmaceutical composition according to claim 13, wherein the pharmaceutical composition is for oral administration, preferably wherein the pharmaceutical composition is a tablet, a capsule, a suspension or a solution.

15. Pharmaceutical composition according to claim 13, wherein the pharmaceutical composition is for intravenous administration, preferably wherein the pharmaceutical composition is a powder for reconstitution or a solution.
